## Europäisches Patentamt

## European Patent Office

## Office européen des brevets

(19)

(11) Veröffentlichungsnummer: **0 068 262**
**B1**

(12)

# EUROPÄISCHE PATENTSCHRIFT

(45) Veröffentlichungstag der Patentschrift:
27.03.85

(21) Anmeldenummer: **82105181.0**

(22) Anmeldetag: **14.06.82**

(51) Int. Cl.⁴: **A 61 D 13/00,** A 61 B 10/00,
A 01 K 29/00

(54) **Vorrichtung zur Bestimmung des Eisprungs bei Haustieren, wie Schweinen, Kühen und Pferden.**

(30) Priorität: **19.06.81 DE 3124121**

(43) Veröffentlichungstag der Anmeldung:
**05.01.83 Patentblatt 83/1**

(45) Bekanntmachung des Hinweises auf die Patenterteilung:
**27.03.85 Patentblatt 85/13**

(84) Benannte Vertragsstaaten:
**AT BE CH DE FR GB IT LI NL SE**

(56) Entgegenhaltungen:
**BE - A - 861 376**
**DE - A - 2 706 444**
**DE - B - 2 115 162**
**FR - A - 2 436 557**
**GB - A - 1 384 254**
**GB - A - 2 077 593**
**US - A - 2 509 825**
**US - A - 3 158 133**
**US - A - 4 151 831**

(73) Patentinhaber: **Rheintechnik Weiland & Kaspar KG,
Grube König, D-6680 Neunkirchen/Saar (DE)**

(72) Erfinder: **Weiland, Werner, Koblenz-Olper-Strasse 172,
D-5413 Bendorf-Sayn (DE)**

(74) Vertreter: **Röbe-Oltmanns, Georg, Dr., Dotzheimer
Strasse 61, D-6200 Wiesbaden (DE)**

## Beschreibung

Die Erfindung betrifft eine Vorrichtung zur Bestimmung des Eisprunges bei Haustieren, wie Schweinen, Kühen und Pferden, die einen in die Scheide des Tieres einführbaren Temperaturfühler aufweist.

Soll bei Haustieren, wie Schweinen, Kühen und Pferden der Eisprung des Tieres bestimmt werden, um es zum richtigen Zeitpunkt zu dekken, so ist es bisher üblich, täglich die Körpertemperatur des Tieres zu messen, wobei der Meßvorgang durch das Einführen eines Thermometers in den After oder die Scheide des Tieres erfolgt. Der Deckvorgang ist dann vorzunehmen, wenn bei dem zu deckenden Tier eine gegenüber der Normaltemperatur erhöhte Körpertemperatur, welche den Eisprung signalisiert, gemessen wird. Diese Temperaturerhöhung ist üblicherweise bei den genannten Haustieren gleich. Unterschiedlich ist jedoch die entsprechende Normaltemperatur der Tiere, welche in gewissen Grenzen schwanken kann. Eine Bestimmung des Eisprungs bei den Tieren ist somit aufwendig, da es nicht nur nötig ist, täglich die Temperatur des zum Deckvorgang anstehenden Tieres zu messen, sondern ebenfalls zur Bestimmung der Temperaturdifferenz die Normalkörpertemperatur des Tieres zu kennen. Besonders aufwendig ist die genannte Methode zur Bestimmung des Eisprunges dann, wenn mehrere Tiere, beispielsweise bei der Zucht zum Decken anstehen.

Aus der DE-B-2 115 162 ist eine Anordnung zum Überwachen des Entbindungsbeginns bei Haustieren mittels eines elektrischen Signalgebers bekannt, bei der eine in den Geburtskanal des Tieres einsetzbare Kapsel mit einem über einen Temperaturfühler ein- und ausschaltbaren Sender sowie einem auf die Sendefrequenz dieses Senders abgestimmten, abseits davon aufgestellten Empfänger als Signalgeber verbunden ist. Wird die Kapsel lange vor Entbindungsbeginn in den Geburtskanal des Muttertieres eingesetzt, bleibt infolge der relativ hohen Temperatur innerhalb des Geburtskanals der Sender der Kapsel und somit auch der Signalgeber ausgeschaltet. Wenn nun die Entbindung beginnt, wird die Kapsel aus dem Geburtskanal herausgestoßen und die Temperatur der Kapsel herabgesetzt. Die Temperaturerniedrigung bewirkt ein Einschalten des Senders, wodurch der elektrische Signalgeber über den zugeordneten Empfänger wirksam wird. In der genannten Auslegeschrift wird ebenfalls ausgeführt, daß der Sender der Kapsel selbstverständlich auch so eingestellt und ausgebildet werden kann, daß er nur bei Erreichen eines vorbestimmten höheren Temperaturwertes, also mit zunehmender Temperatur wirksam wird und nur dann der Empfänger das Signal abgibt. Es wäre somit auch möglich, mit diesem Gerät Fieber bzw. den Eisprung bei Tieren anzuzeigen.

Der Nachteil der in der genannten Auslegeschrift dargestellten Erfindung besteht darin, daß mit dem in der Kapsel befindlichen Meßsystem nur eine bestimmte Temperatur analysiert werden kann, hingegen keine Temperaturdifferenz bestimmbar ist, was insbesondere für die einfache und sichere Angabe des Vorliegens des Eisprungs bei Tieren unerläßlich ist. Das Gerät ist somit ohne einen zusätzlichen Temperaturmesser, mit welchem die Normalkörpertemperatur des Tieres ermittelt werden kann, für eine Bestimmung des Eisprungs ungeeignet. Schließlich ist das Gerät aufgrund der Ausgestaltung der Kapsel, welche in das Tier eingeführt werden muß und zudem aus mehreren Einzelteilen besteht, schwierig zu handhaben. Ein weiterer Nachteil des Gerätes besteht darin, daß nach erfolgtem Eisprung die Kapsel aus dem Tier entfernt werden muß. Bevor die Kapsel in ein anderes Tier eingesetzt werden kann, ist es erforderlich, die Kapsel zu säubern sowie gegebenenfalls auseinanderzunehmen, um einen anderen Temperaturwert einzustellen, bei dem der Temperaturschalter betätigt wird.

Aufgabe der Erfindung ist es, eine Vorrichtung zur einfachen, sicheren Bestimmung des Eisprunges bei Haustieren, wie Schweinen, Kühen und Pferden zu schaffen, welche zudem einfach und sicher zu handhaben ist.

Diese Aufgabe wird bei einer Vorrichtung der eingangs genannten Art dadurch gelöst, daß der Temperaturfühler Temperaturdifferenzen in Richtung steigender Temperaturen ermittelt und bei einer bestimmten Temperaturdifferenz einen Signalgeber betätigt.

Im Gegensatz zur bisher üblichen Temperaturmessung mit einem Thermometer im After oder in der Scheides des Tieres bietet die erfindungsgemäße Temperaturfühlung in der Scheide des Tieres den Vorteil, daß der Temperaturfühler über den Zeitraum, in dem mit dem Eisprung gerechnet wird, im Tier verbleiben kann. Es werden somit von vornherein eventuelle durch das bisherige tägliche neue Einführen des Thermometers in den After oder in die Scheide des Tieres auftretende Meßfehler eliminiert.

Gemäß der Erfindung werden mit dem Temperaturfühler Temperaturdifferenzen ermittelt, da diese ausschließlich für die Bestimmung des Eisprunges aussagekräftig sind. Der Einsatz der Vorrichtung erfolgt derart, daß dann, wenn die Körpertemperatur des Tieres noch der Normaltemperatur entspricht, der Temperaturfühler in die Scheide eingeführt wird und die ermittelte normale Körpertemperatur einen Basiswert zugeordnet bekommt. Zu diesem Basiswert läßt sich mit der Vorrichtung ein Referenzwert einstellen, der in bezug zum Basiswert einer solchen Erhöhung der Körpertemperatur des Tieres entspricht, die Rückschlüsse auf den Eisprung zuläßt.

Vorteilhaft ist es, die Temperaturdifferenz durch einen im Temperaturfühler angeordneten temperaturabhängigen Widerstand zu bestimmen.

Gemäß einer besonderen Ausführungsform der Erfindung sind die aus dem Tier herausführenden Meßleitungen als eine Gesamtleitung ausgebildet. Um ein einfaches Einführen der Leitung in die Scheide des Tieres zu gewährleisten, sollte der Teil der Leitung, welcher in die Scheide des Tieres eingeführt wird, gegenüber dem verbleibenden Teil der Leitung eine geringere Flexibilität aufweisen. Es ist somit gewährleistet, daß zum einen der Temperaturfühler sicher in das Tier eingeführt bzw. aus dem Tier herausgezogen werden kann, zum anderen der aus dem Tier herausragende Teil der Leitung aufgrund dessen Flexibilität das Tier, insbesondere in dessen Schwanzbereich, nicht stört.

Wird die erfindungsgemäße Vorrichtung bei einem Tier eingesetzt, welches sich im Stall von Natur aus ruhig verhält, so kann der Signalgeber an einem Aufstellungsort im Stall, beispielsweise an den Trennwänden der einzelnen Boxen angebracht werden und vorzugsweise mit einem Sender über eine Leitung verbunden sein. Wird die Vorrichtung jedoch bei einem Tier eingesetzt, welches sich im Stall erfahrungsgemäß unruhig verhält oder im Freien aufhält, so müssen Signalgeber und Sender am Tier, vorzugsweise auf dessen Rücken; unter dessen Bauch oder an dessen Hals angebracht werden. Erreicht den Signalgeber über den Temperaturfühler die Information, daß eine Temperaturdifferenz ermittelt wurde, welche auf das Vorliegen des Eisprunges schließen läßt, so wird dieses Signal an den Sender weitergegeben. Es ist vorgesehen, daß der Signalgeber, wenn möglich auch der Sender in der Nähe des Tieres steht, gegebenenfalls ist dem Sender auch eine Einheit vorgeschaltet, welche mehrere Anschlußmöglichkeiten für Vorrichtungen zur Bestimmung des Eisprunges aufweist. Diejenige Person, welche die diesbezügliche Information über das Vorliegen des Eisprunges wünscht, ist mit dem entsprechenden Empfangsgerät ausgestattet und kann somit ihren Tagesablauf unabhängig gestalten.

Ebenfalls der Eintritt der Geburt ist bei Haustieren, wie Schweinen, Kühen und Pferden mit einer Erhöhung der Körpertemperatur des Tiers verbunden. Analog dem Vorgang beim Eisprung ist auch beim Eintritt der Geburt die betreffende Temperatur bei den genannten Tieren verschieden, ungefähr gleichbleibend ist die jeweilige Temperaturdifferenz zwischen der Normaltemperatur des Tieres und der dem Eintritt der Geburt zugeordneten Temperatur. Die erfindungsgemäße Vorrichtung ist demnach nicht nur zur Bestimmung des Eisprunges sondern ebenfalls zur Bestimmung des Eintritts der Geburt geeignet. Das genaue Kennen des Zeitpunktes des Eintrittes der Geburt bei den genannten Haustieren ist deshalb von Wichtigkeit, um bei eventuellen Komplikationen während des Geburtsvorganges helfend eingreifen zu können.

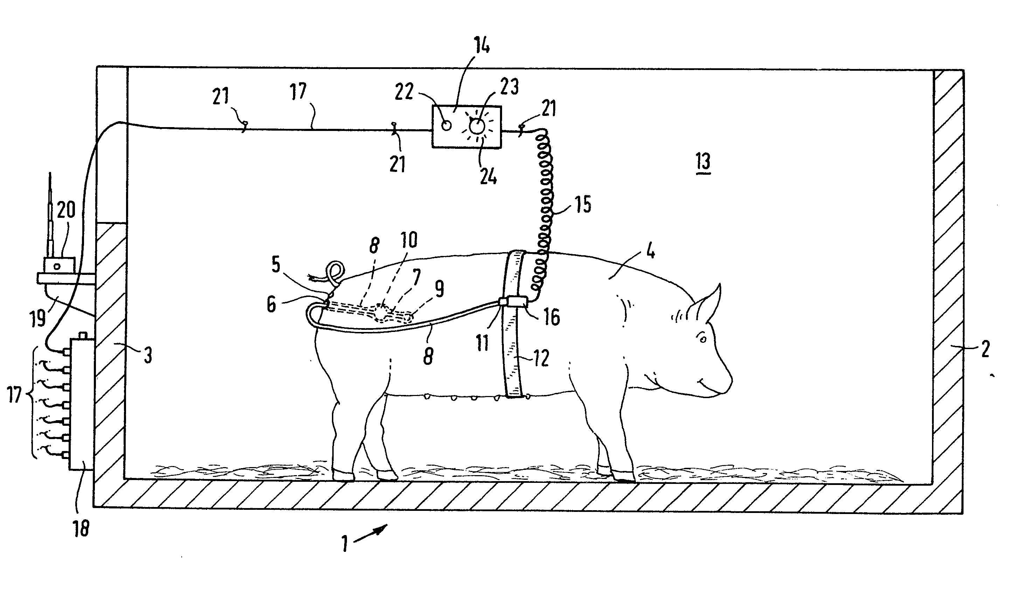

Weitere Merkmale der Erfindung sind in der Beschreibung der Figur und in den Unteransprüchen dargestellt.

Die Figur zeigt eine Seitenansicht in eine Schweinebox, in der sich ein Schwein mit der in die Scheide eingeführten erfindungsgemäßen Vorrichtung befindet.

Mit 1 ist die Box als solche bezeichnet, sie weist ein Frontteil 2 sowie eine Rückwand 3 auf. Die Breite der Box 1 ist so bemessen, daß das in der Box 1 befindliche Schwein 4 sich nicht innerhalb dieser drehen kann.

Durch den unter dem After 5 liegenden Scheideneingang 6 ist in die Scheide 7 ein Teil einer biegsamen Leitung 8 eingeführt, welche an ihrem scheidenseitigen Ende einen Temperaturfühler 9 aufnimmt. Um zu verhindern, daß die Leitung 8 ungewollt aus der Scheide 7 gleitet, weist das in die Scheide 7 des Schweines 4 eingeführte Teil der Leitung 8 eine Verdickung in Form einer aufgesteckten Gummikugel 10 auf. Die Leitung 8 ist mit einer Ummantelung, beispielsweise aus Gummi, versehen, welche vom Kot bzw. vom Urin des Tieres nicht angegriffen wird. Das dem Temperaturfühler 9 angewandte Ende der Leitung 8 weist an dessen Ende einen Stecker 11 auf. Der Stecker 11 ist mit Hilfe eines Gurtes 12 seitlich am Schwein 4 befestigt. An einer Seitenwand 13 der Box 1 ist ein Signalgeber 14 angeordnet. Die Verbindung von Signalgeber 14 und Leitung 8 erfolgt über eine flexible Leitung 15, welche an ihrem, dem Signalgeber 14 abgewandten Ende einen Stecker 16 zur Verbindung mit dem Stecker 11 aufweist. Eine weitere Verbindung ist auf Grund der Leitung 17 zwischen dem Signalgeber 14 und einer Steckerleiste 18 hergestellt. Mit dieser Steckerleiste 18 können mehrere Signalgeber 14 über entsprechende Leitungen 17 verbunden werden. Von der Steckerleiste 18 erfolgt über eine weitere Leitung 19 der Anschluß zum Sender 20. Insbesondere Teile der innerhalb der Box 1 verlaufenden Leitungen 15 und 17 können mit Hilfe von Befestigungsmitteln 21 an der entsprechenden Seitenwand 13 in der Box 1 fixiert sein.

Soll bei dem Schwein 4 der Eisprung bestimmt werden, so wird bereits dann, wenn die Körpertemperatur des Schweines 4 noch dessen Normaltemperatur entspricht, die biegsame Leitung 8 in die Scheide 7 eingeführt. Um ein einfaches Einführen der Leitung in die Scheide des Schweines zu gewährleisten, sollte der Teil der Leitung, welcher in die Scheide des Schweines eingeführt wird, gegenüber dem verbleibenden Teil der Leitung eine geringere Flexibilität aufweisen.

Ist die Leitung 8 mit dem Temperaturfühler 9 in die Scheide 7 des Schweines 4 eingeführt, so kann durch die Abgleichung einer entsprechenden Schaltung im Signalgeber dieser Normaltemperatur ein Basiswert, beispielsweise Null zugewiesen werden. Der Basiswert kann dadurch ermittelt werden, daß mehrere Stromkreise mit variablen Widerständen solange durchgeschaltet werden, bis ein Widerstand eines Stromkreises exakt auf einen, eine definierte Temperatur repräsentierenden NTC-Widerstand abgestimmt ist. Das Durchschalten als solches erfolgt über einen drehbaren Schalter 23, im Fall der Abstimmung der beiden Widerstände leuchtet eine auf dem Signalgeber 14 angebrachte Lampe 22 auf. Für die Bestimmung des Basiswertes ist demnach das Kennen der Normaltemperatur des Tieres überflüssig.

Die gegenüber der Normaltemperatur erhöhte Temperatur, bei der der Eisprung stattfindet, stellt einen Erfahrungswert dar. Entsprechend dieser Temperaturdifferenz wird der Schalter 23 auf einer Skala 24 um Temperaturdifferenzen repräsentierende Einheiten in Richtung steigender Werte verdreht. Dies hat zur Folge, daß die auf dem Signalgeber 14 angeordnete Lampe 22 erlischt. Hat der Eisprung beim Tier stattgefunden, wurde somit die eingestellte Temperaturdifferenz überschritten, leuchtet die auf dem Signalgeber 14 angeordnete Lampe 22 wieder auf, weiterhin wird das Signal über die Leitung 17, über die Steckerleiste 18 und die Leitung 19 dem Sender 20 zugeführt. Das ausgestrahlte Signal wird von einem nicht dargestellten Empfänger aufgenommen und löst einen Alarm aus. Auf Grund des Alarmes, sowie der auf dem Signalgeber 14 brennenden Lampe 22 ist es möglich, das Tier herauszufinden, welches zum Decken ansteht.

Die Information, daß bei einem Tier der genannten Art ein Eisprung stattgefunden hat, kann nicht nur über das dargestellte Sendesystem erfolgen, sondern kann ganz allgemein dadurch möglich sein, daß der Signalgeber 14 nur mit einem akustischen und/oder optischen Informationsträger verbunden ist.

## Patentansprüche

1. Vorrichtung zur Bestimmung des Eisprunges bei Haustieren, wie Schweinen, Kühen und Pferden, die einen in die Scheide (7) des Tieres (4) einführbaren Temperaturfühler (9) aufweist, dadurch gekennzeichnet, daß der Temperaturfühler (9) Temperaturdifferenzen in Richtung steigender Temperaturen ermittelt und bei einer bestimmten Temperaturdifferenz einen Signalgeber (14) betätigt.

2. Vorrichtung nach Anspruch 1, dadurch gekennzeichnet, daß die Bestimmung der Temperaturdifferenz durch einen im Temperaturfühler (9) angeordneten temperaturabhängigen Widerstand erfolgt.

3. Vorrichtung nach Anspruch 2, dadurch gekennzeichnet, daß im Signalgeber (14) mehrere Schaltkreise mit Widerständen unterschiedlicher Größe angeordnet sind, wobei jeweils einer dieser Widerstände auf den im Temperaturfühler (9) angeordneten, temperaturabhängigen Widerstand abgleichbar ist.

4. Vorrichtung nach einem oder mehreren der Ansprüche 1 bis 3, dadurch gekennzeichnet, daß der Signalgeber (14) über eine Leitung (8, 15) mit dem Temperaturfühler (9) verbunden ist.

5. Vorrichtung nach Anspruch 4, dadurch gekennzeichnet, daß die Leitung (8) aus einem säurefesten, insbesondere gegen Fäkalien resistenten Material, wie Gummi, besteht.

6. Vorrichtung nach einem oder mehreren der Ansprüche 1 bis 5, dadurch gekennzeichnet, daß der in die Scheide (7) des Tieres (4) einführbare Teil der Leitung (8) eine Verdickung, beispielsweise in Form einer aufgesteckten Gummikugel (10) aufweist.

7. Vorrichtung nach einem oder mehreren der Ansprüche 1 bis 6, dadurch gekennzeichnet, daß der Signalegeber (14) an der Box (1), insbesondere an einer Seitenwand (13) der Box (1) angeordnet ist.

8. Vorrichtung nach einem oder mehreren der Ansprüche 1 bis 7, dadurch gekennzeichnet, daß der Signalgeber (14) über eine Leitung (17, 19) mit einem Sender (20) verbunden ist.

9. Vorrichtung nach einem oder mehreren der Ansprüche 1 bis 8, dadurch gekennzeichnet, daß der Signalgeber und der Sender am Tier, vorzugsweise auf dessen Rücken, unter dessen Bauch oder an dessen Hals befestigbar sind.

## Claims

1. Device for determining ovulation in domestic animals such as pigs, cows and horses, which comprises a temperature sensor (9) insertable in the vagina (7) of the animal (4), characterised in that the tmperature sensor (9) ascertains temperature difference in the sense of rising temperatures and, at a specific temperature difference, actuates a signal emitter (14).

2. Device according to claim 1, characterised in that the determining of the temperature difference is effected by a temperature-dependent resistor situated in the temperature sensor (9).

3. Device according to claim 2, characterised in that a plurality of circuits with resistors of varying value are arranged in the signal emitter (14), and in each case one of these resistors is adjustable to the temperature-dependent resistor arranged in the temperature sensor (9).

4. Device according to one or more of claims 1 to 3, characterised in that the signal emitter (14) is connected to the temperature sensor (9) via a cable (8, 15).

5. Device according to claim 4, characterised in that the cable (8) is made of an acid-resistant material, especially a material resistant to faeces, such as rubber.

6. Device according to one or more of claims 1 to 5, characterised in that that part of the cable (8) which is insertable into the vagina (7) of the animal (4) comprises a thickened protion, for example in the form of a rubber ball (10) arranged thereon.

7. Device according to one or more of claims 1 to 6, characterised in that the signal emitter (14) is arranged on the animal's box (1), especially on one side wall (13) of the box (1).

8. Device according to one or more of claims 1 to 7, characterised in that the signal emitter (14) is connected by means of a cable (17, 19) to a transmitter (20).

9. Device according to one or more of claims 1 to 8, characterised in that the signal emitter and the transmitter are securable to the animal, preferably on its back, under its belly, or to its neck.

## Revendications

1. Dispositif pour la détermination de la période d'ovulation chez des animaux domestiques tels que truies, vaches et juments, qui comporte un détecteur de température (9) introductible dans le vagin (7) de l'animal (4), dispositif caractérisé en ce que le détecteur de température (9) détermine des différences de température dans le sens de températures croissantes et que, pour une différence de température déterminée, il actionne un émetteur de signal (14).

2. Dispositif selon la revendication 1, caractérisé en ce que la détermination de la différence de température s'effectue au moyen d'une résistance variant avec la température, disposée dans le détecteur de température (9).

3. Dispositif selon la revendication 2, caractérisé en ce que dans l'émetteur de signal (14), se trouvent plusieurs circuits de connexion avec des résistances de différentes valeurs, une de ces résistances pouvant toujours être étalonnée par rapport à la résistance dépendant de la température disposée dans le détecteur de température (9).

4. Dispositif selon l'une quelconque des revendications 1 à 3, caractérisé en ce que l'émetteur de signal (14) est relié au détecteur de température (9) au moyen d'un conducteur électrique (8, 15).

5. Dispositif selon la revendication 4, caractérisé en ce que le conducteur électrique (8) est fait d'une matière résistant aux acides, notamment aux matières fécales, telle que du caoutchouc.

6. Dispositif selon l'un quelconque des revendications 1 à 5, caractérisé en ce que la partie du conducteur électrique (8) destinée à être insérée dans le vagin (7) de l'animal (4), présente un point de surépaisseur, par exemple en forme d'une boule de caoutchouc (10).

7. Dispositif selon l'une quelconque des revendications 1 à 6, caractérisé en ce que l'émetteur de signal (14) est disposé dans le box de stabulation (1), et notamment fixé à une paroi latérale (13) de ce box (1).

8. Dispositif selon l'une quelconque des revendications 1 à 7, caractérisé en ce que l'émetteur de signal (14) est relié par une canalisation électrique (17, 19) à un indicateur de signal (20).

9. Dispositif selon l'une quelconque des revendications 1 à 8, caractérisé en ce que l'émetteur de signal et l'indicateur de signal peuvent être fixés sur l'animal, de préférence sur le dos, le cou ou sous le ventre.